# EUROPEAN PATENT APPLICATION

(11) **EP 2 255 769 A1**
(43) Date of publication of application: **01.12.2010**
(21) Application number: 09161036.0
(22) Date of filing: 25.05.2009
(51) Int. Cl.: A61F 13/56, A61F 13/62

(54) **Absorbent article with elastic fastening tab and method of manufacturing said article**

(71) Applicant: 3M Innovative Properties Company, St. Paul MI 55133-3427 (US)
(72) Inventor: Petersen, Johann, 41516 Grevenbroich (DE); Kitzer, Peter, 6101 BZ Echt (NL)
(74) Representative: Wilhelm, Stefan

(57) **Abstract**

The present invention provides an absorbent article (1) comprising a top sheet (90) and a back sheet (80) and two opposite longitudinal edges (62) extending along the extension of the absorbent article (1) in machine direction , the absorbent article comprising at least one strip-shaped fastening tab (10,10a) for securing said article on a wearer during use, wherein said fastening tab (10,10a)
- comprises a fastening portion (30) through which it is attached to the absorbent article (1) and a gripping portion (20) which is opposite to the fastening portion (30),
- is elastically extendable or stretch-activatable to become elastically extendable, respectively,
- is not folded upon itself in the non-extended state, and
wherein less than 25 % of the extension of the fastening tab in CD in its non-extended state protrudes beyond or undershoots, respectively, the longitudinal edge (62) of the diaper.

## Description

### Field of the invention

The present invention is directed to absorbent articles such as disposable diapers having a fastening tab, which is not folded onto itself in the non-extended state and which is elastically extendable or stretch-activatable to become elastically extendable, respectively. The invention furthermore refers to methods of manufacturing said absorbent articles.

### Background of the invention

Disposable products, such as diapers, are widely used not only in the sector of child care but also for adults suffering from incontinence. These disposable products are typically re-closable by fastening tabs securing the absorbent article to the body of the wearer.

The fastening tabs preferably are elastically extendable to increase the wearer's comfort and to improve the anchoring of the absorbent article when the wearer moves. EP 0,704,196 discloses, for example, an elastic fastening tape for a disposable diaper comprising an elastic tape section secured at least at its ends to the fastening tape to bridge a central portion thereof. The elastic portion comprises an elastic film or laminate or a stretch-activatable film or laminate which can be rendered elastic, for example, by stretching. The central portion of the fastening tape is rendered elastic by passing it between a zigzag-shaped gap between two dies. Elastic fastening tabs are also disclosed, for example, in US 5,620,780, EP 0,521,883 and EP 1,066,008.

The fastening tabs are commonly attached to the back sheet of the rear portion of the absorbent article, e. g. a disposable diaper, so that the tabs can be folded over onto the back sheet on the front portion of the diaper in order to secure the diaper to the body of the wearer. As is illustrated, for example in EP 0,704,196 the fastening tab is typically attached to the diaper at one of its end portions (also referred to as fastening portions or manufacturer's end, respectively) whereas the other end portion (also referred to as the gripping portion or the user's end, respectively) and a major part of the intermediate portion in-between protrude over the adjacent longitudinal edge of the diaper or diaper chassis, respectively, to provide for enough length of the tab so that it can be applied to the front portion of the diaper when applied to the wearer.

During manufacturing the portion of the fastening tab protruding beyond the adjacent longitudinal edge is folded around such edge and temporarily attached to the top sheet of the diaper as is illustrated, for example, in EP 0,704,196. This is required to avoid flagging of the protruding portion of the tape tab during manufacturing which may result in damaging the fastening tab or even rupturing it from the diaper during manufacturing. Fastening tabs designed to avoid the flagging effect are disclosed, for example, in EP 1,437,993 and EP 0, 321,232. The temporary attachment of the fastening tab to the top sheet of the diaper is typically achieved by engaging a mechanical fastening element of the fastening tab with a complementary, for example non-woven surface of the top sheet or by using an adhesive attachment means such as, for example, an open pressure sensitive adhesive area which can be arranged on the fastening tab or the top sheet, respectively. It is also possible to temporarily fix the fastening tab on the top sheet by hot-melt adhesive spots or by ultrasonic bonding points. The temporary attachment of the fastening tab is broken by the user when gripping the gripping portion and attaching the fastener tab to the back sheet on the front portion of the diaper.

Folding over the fastening tab during manufacturing is an additional manufacturing step which may slow down the diaper line speed and create additional costs. Also, since the temporary attachment to the top sheet of the diaper needs to be easily breakable and thus relatively weak, the fastening tab may "pop open" so that the flagging effect may not be reliably prevented.

It has also been suggested, for example, in EP 0,482,383 and in EP 1,214,034 to fold the fastening tabs in the non-extended state upon themselves to provide a so-called "z-fold" arrangement. Such fastening tab may be arranged on the back sheet so that it does not protrude the longitudinal edge of the diaper during the manufacturing phase. When applying the diaper to a wearer's body the user's end of the z-folded tab is gripped by the user thereby unfolding z-folded tab and providing enough length to attach it to the front portion of the diaper. The z-folded fastening tabs of EP '383 and EP '034 exhibit two folds each.

US 6,544,242 and US 6,036,066 disclose pull-up diapers comprising an elastically or plastically extendable disposal tab, respectively. The disposal tabs are attached to the back sheet on the front portion of the diaper so that they are arranged essentially normal to the outer edge of the elastic waist area of the diaper whereas fastening tabs are generally oriented in the direction of the waist area. The disposal tab furthermore comprises a gripping portion and an intermediate extendable portion which are temporarily attached to the diaper. After use, the soiled diaper bearing the exudates is rolled up and the disposal tape is gripped by the user, extended and folded around the waist portion to secure the diaper in the rolled up state and avoid spillage of the exudates. The disposal tabs disclosed in US '242 and US '066 are arranged in some distance from the outer edge of the elastic waist area of the diaper so that they require excessive stretching when applied to the rolled-up, soiled diaper.

It was an object of the present invention to provide a disposable article and, in particular, a diaper or a side panel, respectively, comprising at least one elastic or elastically activatable fastening tab, respectively, which does not exhibit the shortcomings of the prior art constructions or shows them to a lower extent only, respectively. It was another object of the present invention to provide such a disposable article or a side panel, respectively, comprising at least one elastic fastening tab wherein the fastening tab exhibits a simple construction without any fold in the non-extended state. It was another object of the present invention to provide such a disposable article and, in particular, a diaper or a side panel, respectively, comprising at least one elastic fastening tab which can be advantageously manufactured at high production speeds. It was another object of the present invention to provide a method for manufacturing absorbent articles or side panels, respectively, wherein the fastening tabs are arranged on the absorbent articles or side panels, respectively, that flagging is reduced in comparison to conventional manufacturing processes so that ripping off of the fastening tabs is safely avoided. Other objects of the present invention can be taken from the following detailed specification.

### Summary of the invention

The present invention relates in one aspect to an absorbent article comprising a top sheet and a back sheet and two opposite longitudinal edges extending along the extension of the absorbent article in machine direction , the absorbent article comprising at least one strip-shaped fastening tab for securing said article on a wearer during use, wherein
- said fastening tab comprises a fastening portion through which it is attached to the absorbent article and a gripping portion which is opposite to the fastening portion,
- said fastening tab is elastically extendable or stretch-activatable to become elastically extendable, respectively,
- said fastening tab is not folded upon itself in the non-extended state, and
wherein less than 25 % of the extension of the fastening tab in CD in its non-extended state protrudes beyond or undershoots, respectively, the longitudinal edge of the diaper.

The present invention relates in another aspect to an absorbent article comprising a top sheet and a back sheet and two opposite longitudinal edges extending along the extension of the absorbent article in machine direction , the absorbent article comprising at least one strip-shaped fastening tab for securing said article on a wearer during use, wherein
- said fastening tab comprises a fastening portion through which it is attached to the absorbent article and a gripping portion which is opposite to the fastening portion,
- said fastening tab is elastically extendable or stretch-activatable to become elastically extendable, respectively,
- said fastening tab is not folded upon itself in the non-extended state, and wherein the absolute value of the distance between the distal edge of the fastening tab and the longitudinal edge of the diaper is less than 15 mm.

The present invention relates in yet another aspect to a side panel comprising a top sheet and a back sheet, a distal longitudinal edge and a proximal longitudinal edge being attachable to the chassis of an absorbent article wherein both edges are extending along the extension of the side panel in machine direction, the side panel comprising at least one strip-shaped fastening tab
- which comprises a fastening portion through which it is attached to the side panel and a gripping portion which is opposite to the fastening portion,
- which is elastically extendable or stretch-activatable to become elastically extendable, respectively,
- which is not folded upon itself in the non-extended state, and
wherein less than 25 % of the extension of the fastening tab in CD in its non-extended state protrudes beyond or undershoots, respectively, the distal longitudinal edge of the side panel.

The present invention relates in yet another aspect to a side panel comprising a top sheet and a back sheet, a distal longitudinal edge and a proximal longitudinal edge being attachable to the chassis of an absorbent article wherein both edges are extending along the extension of the side panel in machine direction, the side panel comprising at least one strip-shaped fastening tab
- which comprises a fastening portion through which it is attached to the side panel and a gripping portion which is opposite to the fastening portion,
- which is elastically extendable or stretch-activatable to become elastically extendable, respectively,
- which is not folded upon itself in the non-extended state, and
wherein the absolute value of the distance between the distal edge of the fastening tab and the distal longitudinal edge of the side panel is less than 15 mm.

The present invention relates yet in another aspect to a continuous web comprising a sequence of side panels according to the invention. If desirable, such web may be stored in the form of a stable roll from which individual side panels may be cut.

The present invention furthermore relates in yet another aspect to a method of manufacturing absorbent articles comprising the steps of
(i) providing a web of absorbent articles comprising a top sheet and a back sheet and two opposite longitudinal edges extending along the extension of the absorbent article in machine direction,
(ii) providing a sequence of individual fastening tabs which each
   - comprises a fastening portion through which it can be attached to the absorbent article and a gripping portion which is opposite to the fastening portion,
   - is elastically extendable or stretch-activatable to become elastically extendable, respectively,
   - is not folded upon itself in the non-extended state,
(iii) applying at least one fastening tabs to an absorbent article of the web so that less than 25 % of the extension of the fastening tab in CD protrudes in its non-extended state beyond the longitudinal edge of the absorbent article, and
(iv) applying CD cuts to the web of absorbent articles to provide a sequence of individual absorbent articles each exhibiting at least one individual fastening tab.

The present invention furthermore relates in yet another aspect to a method of manufacturing side panels comprising the steps of
(i) providing a web of side panels comprising a top sheet and a back sheet, a distal longitudinal edge and a proximal longitudinal edge being attachable to the chassis of an absorbent article, with both such edges extending along the extension of the side panel in machine direction,
(ii) providing a sequence of individual fastening tabs each of which
   - comprises a fastening portion through which it can be attached to the side panel and a gripping portion which is opposite to the fastening portion,
   - is elastically extendable or stretch-activatable to become elastically extendable, respectively,
   - is not folded upon itself in the non-extended state,
(iii) applying at least one fastening tab to a side panels of the web so that less than 25 % of the extension of the fastening tab in CD protrudes in its non-extended state beyond the distal longitudinal edge of the side panel, and
(iv) applying CD cuts to the web of side panels to provide a sequence of individual side panels each exhibiting at least one individual fastening tab.

### Brief description of the drawings

Fig. 1a is a perspective view of a first embodiment of a side panel of the invention comprising a fastening tab being in a single-sided arrangement on the back sheet of the side panel so that the distal edge of the gripping portion of the side panel is arranged adjacent to the distal longitudinal edge of the side panel.
Fig. 1b is a cross-sectional view of the side panel of Fig. 1a along the line 1b-1b indicated in Fig. 1a.
Fig. 2a is a perspective view of the side panel of Fig. 1a showing the fastening tab in an extended state.
Fig. 2b is a cross-sectional view of the side panel of Fig. 2a along the line 2b-2b indicated in Fig. 2a.
Fig. 3a is a perspective view of a further embodiment of a side panel of the invention comprising a fastening tab 10 in a single-sided arrangement on the back sheet of the side panel whereby at least part of the gripping portion of the fastening tab is exposed through a distally open cut-out area in the side panel which is indicated by the dotted line underneath the fastening tab.
Fig. 3b is a cross-sectional view of the side panel of Fig. 3a along the line 3b-3b indicated in Fig. 3a.
Fig. 3c is a perspective view of the side panel of Fig 3a wherein the fastening tab is distally elevated from the back sheet but not extended yet.
Fig. 4a shows a cross-sectional view of a further embodiment of an absorbent article of the invention wherein the fastening portion of the fastening tab is arranged adjacent to the longitudinal edge of the absorbent article.
Fig. 4b is a cross-sectional view of the absorbent article of Fig. 4a where the fastening tab has been opened and is folded over but is not extended yet.
Fig. 5a shows a cross-sectional view of a further embodiment of an absorbent article of the invention comprising a fastening tab being in a single-sided arrangement on the top sheet of the absorbent article so that the distal edge of the gripping portion of the side panel is arranged adjacent to a longitudinal edge of the absorbent article.
Fig. 5b is a cross-sectional view of the absorbent article of Fig. 5a showing the fastening tape tab in an extended state.
Fig. 6a shows a cross-sectional view of a further embodiment of an absorbent article of the invention which is similar to the embodiment of Fig. 4a with the exception that the fastening tab is attached to the top sheet of the absorbent article.
Fig. 6b is a cross-sectional view of the absorbent article of Fig. 6a showing the fastening tape tab in an extended state.
Fig. 7a shows a perspective view of a diaper of the invention having an hourglass shape in an unfolded state prior to applying it to a wearer. The fastening tape tabs are in a single sided arrangement on the back sheet of the rear portion of the diaper so that in the non-extended state shown only the griping portion of the tab protrudes beyond the longitudinal edge of the diaper.
Fig. 7b is a perspective view of the diaper of Fig. 7a in the state as applied to a wearer.
Fig. 7c shows a perspective view of another diaper having an essentially rectangular chassis with two side panels attached to the rear portion of the chassis. The fastening tabs are in a single-side configuration on the back sheet of the rear portion of the diaper as in Fig. 7a.
Fig. 8a shows a perspective view of a web of side panels which is cut along cut-lines into MD and CD, respectively, into a sequence of pairs of complementary left and right sides panels.
Fig. 8b and 8c are cross sectional views of figure 8a along the lines 8b-8b and 8c-8c, respectively, indicated in Fig. 8a.
Fig. 9a shows a perspective of a web of side panels which are cut along MD and CD cut lines, respectively, to provide individual side panels extending essentially over the extension of the absorbent article in MD. Each side panel exhibits enlarged areas at its upper and lower end in MD which provide when applied to the diaper chassis so-called "ears" in the rear and front portion of the diaper, respectively.
Fig. 9b shows the design of a diaper comprising an essentially rectangular chassis 1b and a pair of side panels 1a obtained in the process illustrated in Fig. 9a.
Fig. 10a shows a top view of a web of side panels illustrating a method of manufacturing individual side panels. The method comprises die-cutting two adjacent cut-out areas, covering the cut-out areas with a fastening pad comprising two fastening tabs and applying appropriate cuts in MD and CD, respectively.
Fig. 10b shows a cross-sectional view of the web of side panels along the line 10b-10b indicated in Fig. 10a.
Fig. 11a is a perspective view of another embodiment of a side panel of the invention comprising a fastening tab being in a sandwich arrangement between the top sheet and the back sheet. The gripping portion is protruding beyond the distal longitudinal edge of the side panel.
Fig. 11b is a cross-sectional view of the side panel of Fig. 11a along the line 11b-11b indicated in Fig. 11a.
Fig. 12a is a perspective view of another embodiment of a side panel of the invention comprising a fastening tab being in a sandwich arrangement between the top sheet and the back sheet. A distally open cut-out portion extends through both the top sheet and the back sheet to expose a part of the gripping portion extending proximally from the distal edge of the side panel.
Fig. 12b is a cross-sectional view of the side panel of Fig. 12a along the line 12b-12b indicated in Fig. 12a.

### Detailed description of the invention

As used above and below, the term "absorbent article" refers to articles which are placed against or in proximity to the body of a wearer to absorb and contain the various exudates discharged from the body. The absorbent article preferably is disposable, i.e. it is intended to be disposed of after single use and not intended to be laundered or otherwise restored or used.

A preferred embodiment of the absorbent article referred to in the present invention is a diaper which may be used by infants or incontinent adults, for example. During use, such diapers are typically drawn up between the legs and fastened around the waist of the wearer.

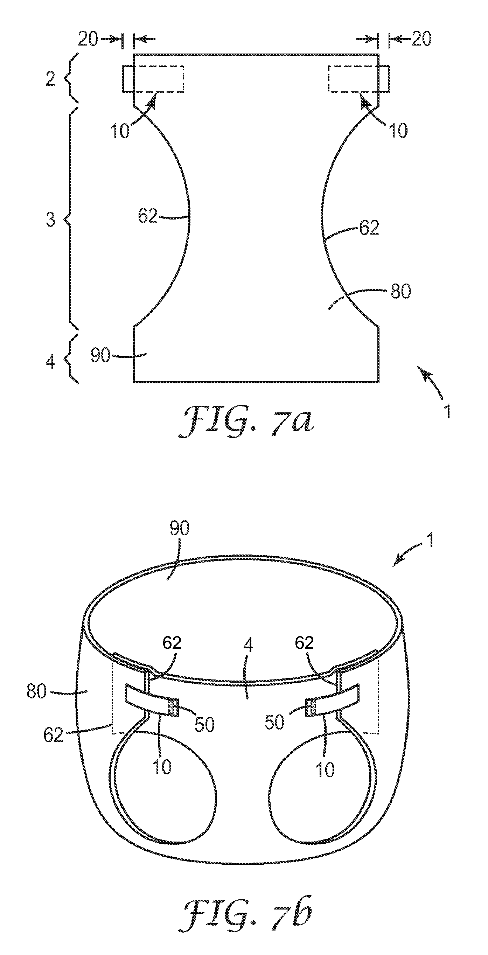

The absorbent article may be of an hour-glass shape as is illustrated in Fig.'s 7a and 7b below. In such design the rear portion of the diaper (i.e. the portion facing the rear of the wearer when the diaper is attached) and the waist portion of the diaper (i.e. the portion facing the waist of the wearer when the diaper is attached) are wider in CD as compared to the crotch area between such rear portion and waist portion. This arrangement typically results in an overlap of the rear area and the waist area when applying the diaper to the wearer as can be seen in Fig. 7b. The rear portion and the waist portion of the diaper are connected by the intermediate crotch portion as is shown, for example, in Fig.'s 7a, and 9b. When applying the diaper to a wearer the crotch portion forms leg openings as can be seen from the schematic representation of Fig. 7b.

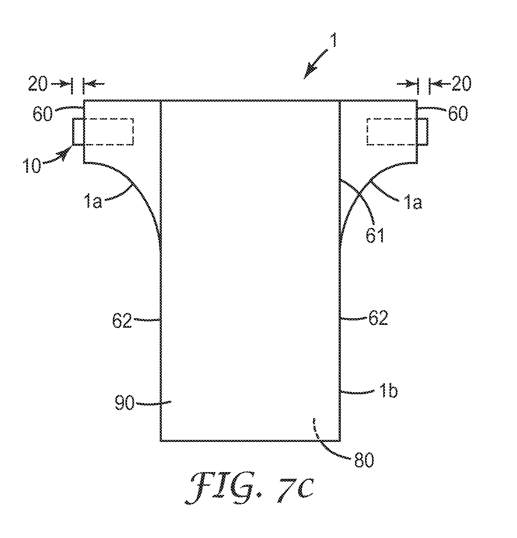

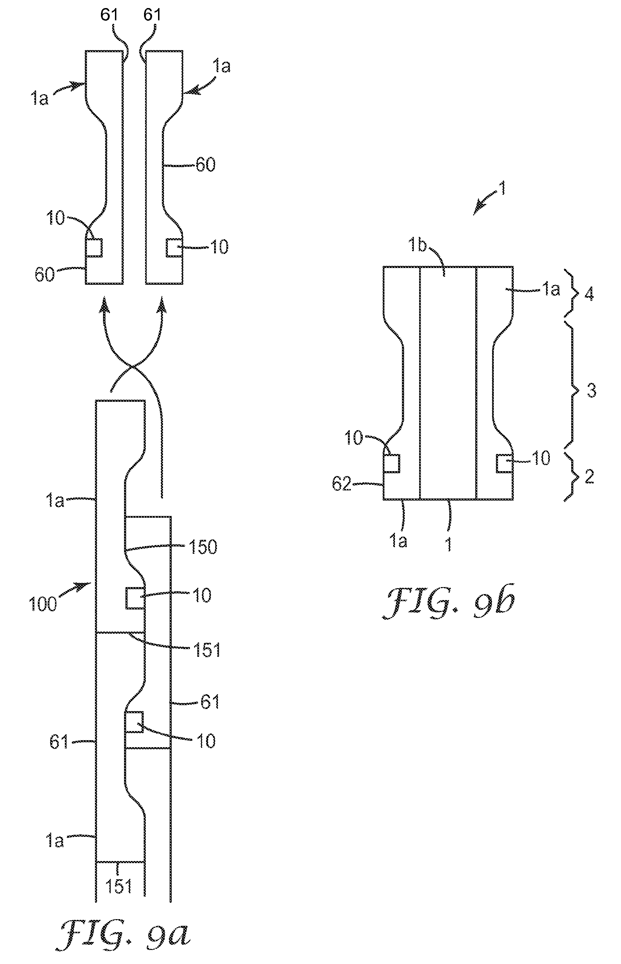

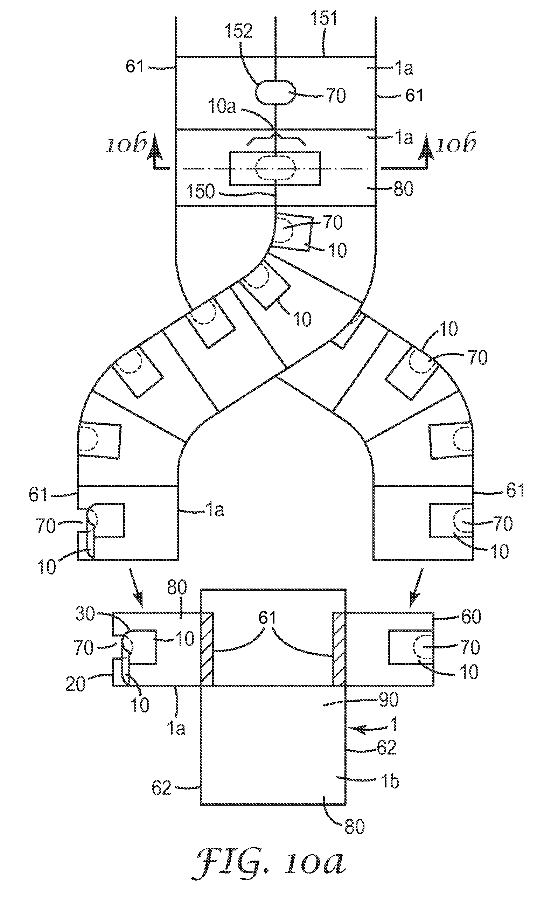

It is also possible that the diaper comprises an essentially rectangular chassis to which at least one side panel is applied in the rear portion and/or the waist portion of the chassis, respectively. Usually, at least one side panel is applied through its proximal longitudinal edge (or the area close to such edge, respectively) along each of the longitudinal edges (or the areas close to such edges) of the rear portion of the chassis of the diaper as is illustrated, for example, in the diaper designs of Fig. 7c or 10a, respectively. It is also possible to attach at least one side panel to both the rear portion and the waist portion of the diaper. In a preferred embodiment, a pair of side panels which may be, for example, essentially rectangular or trapezoidal shaped, can be attached to both the rear portion and the waist portion, respectively, of the chassis of the diaper. It is also possible, for example, to provide side panels which extend over essentially the full length of the diaper in MD as is illustrated, for example, in Fig. 9a; the side panels of Fig. 9a provide when attached to the chassis of a diaper so-called "ear" sections both in the rear and front portions of the diaper, respectively (cf. Fig. 9b). The side panels do not need to be symmetrical like those shown in Fig. 9a but it is also possible, for example, that the extension of the side panels in CD is wider in the rear portion as compared to the CD extension in the waist portion.

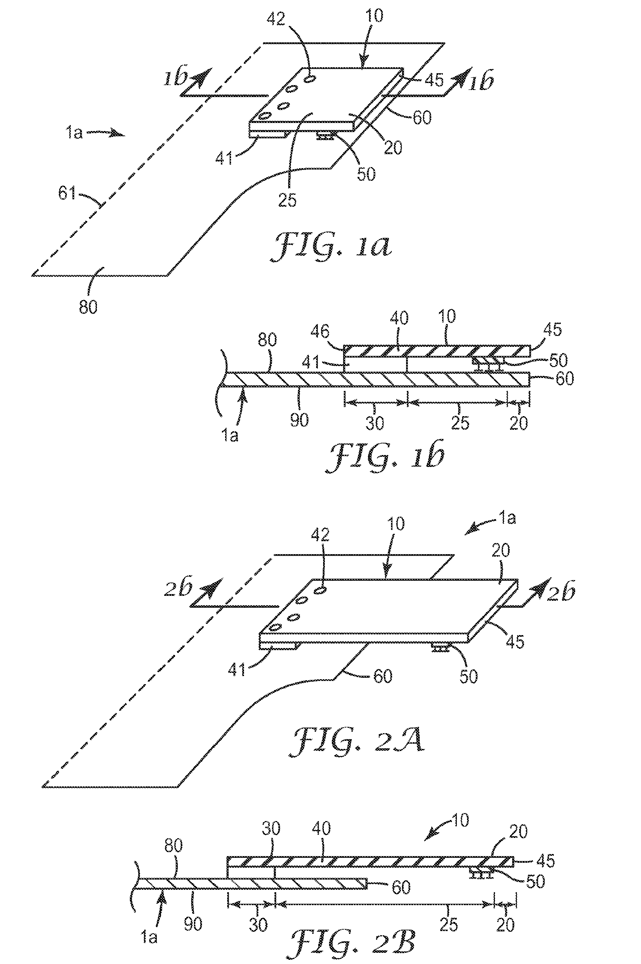

The side panels are generally shaped so that a good fit and an ergonomic shape of the absorbent article is obtained. The edges of the side panels may be curved as is exemplified in Fig.1a or Fig. 7c.

As used above and below, the term machine direction (MD) of the absorbent article, the side panel, or the fastening tab, respectively, denotes the direction of the running, continuous web of absorbent articles or of the web of side panels, respectively, as is shown, for example, in Fig.'s 8a, 9a and 10a. Fastening tabs are usually cut from a web running in MD, as well, so that the same terminology is used for fastening tabs. For a diaper, MD thus corresponds to the direction of the longitudinal extension of the diaper which is essentially normal to the rear portion 2 and the waist portion 4 of the diaper, respectively. The cross direction (CD) is the direction normal to MD and corresponds, for example, to the direction of the extension of the rear portion 2. Likewise, the direction of the longitudinal extension of a side panel is referred to as MD. For a fastening tab, CD corresponds to the direction of the longitudinal extension from the fastening portion to the gripping portion.

A part of the absorbent article, the side panel or the fastening tab which is arranged "proximally" is located in use closer to the wearer's body, or is closer to the center line of the absorbent article in MD, respectively. Correspondingly, a part of the absorbent article, the side panel or the fastening tab which is arranged "distally" is located in use farther away from the wearer's body, or is farther away from the center line or the absorbent article in MD, respectively. The portion of the fastening tab, for example, arranged adjacent to the longitudinal edge of a diaper, is referred to as being arranged distally whereas the opposite portion of the fastening tab arranged in some distance from such longitudinal edge is arranged proximally.

When a first part of the absorbent article, the side panel or the fastening tab, respectively, is arranged "adjacent" to another second part of the absorbent article, the side panel or the fastening tab, respectively, this is understood above and below to denote that such parts are arranged in proximity of one another whereby a variety of positions is possible. If the distal edge of the fastening tab is essentially parallel and adjacent in CD to the longitudinal edge of the diaper, for example, the orientation and position of the distal edge of the fastening tab essentially matches that of the longitudinal edge of the diaper in CD.

As used above and below, the term "edge" denotes the line where the absorbent article, the side panel or the fastening tab, respectively, end. An absorbent article such as, in particular, a diaper, or a side panel, respectively, exhibit longitudinal edges extending in MD and bordering the absorbent article in CD. The longitudinal edges of a side panel are further characterized as being proximal or distal, respectively, thereby reflecting the orientation of the side panel relative to the absorbent article such as a diaper. The absorbent article or the side panel, respectively, may exhibit cut-out areas to facilitate gripping of the fastening tab(s) as will be disclosed in more detail below. In such cut-out areas the edge of the absorbent article or the side panel, respectively, is formed by the hypothetical, extrapolated edge the absorbent article or the side panel, respectively, exhibited prior to applying the cut-out area(s) and not to the new edge created by applying the cut-out area(s).

The back sheet and the top sheet of the absorbent article or the side panel, respectively, preferably comprise a nonwoven-like material to provide a cloth-like appearance. The non-woven materials of the top sheet and back sheet can be selected independently from each other or they can be the same. The non-woven material of the back sheet is preferably selected so as to mechanically engage with the fastening means on the fastening tab (often referred to as hook-to-back sheet engagement). If desirable, the back sheet and/or the top sheet may comprise other materials such as, for example, polymeric films, wovens or textiles. The back sheet is preferably liquid-impermeable whereas the top sheet is usually liquid-permeable.

The top sheet and the back sheet of the absorbent article or the side panel, respectively, are connected or otherwise associated together in any operable manner. The top sheet can be affixed directly to the back sheet but it is also possible that back sheet and top sheet are connected or joined indirectly with each other by affixing the top sheet to intermediate member(s) which in turn are affixed to the back sheet. The top sheet and the back sheet can be connected or affixed directly to each other in the periphery of the absorbent article or on the side panel, respectively, by, for example, adhesive bonds, sonic bonds, thermal bonds or any other attachment means.

In the central part of the absorbent article the top sheet and the back sheet sandwich an absorbent core which is generally compressible, conformable, non-irritating to the wearer's skin, and capable of absorbing and retaining liquid body exudates. The absorbent core may comprise, for example, hydrophilic fibrous material and high-absorbing materials and can be provided, for example, in the form of an absorbent pad. The absorbent core may comprise a single, integral piece of material, or alternatively, may comprise a plurality of individual separate pieces of material which are operably assembled together.

The absorbent article or the side panel of the present invention, respectively, comprise at least one fastening tab for securing said article on a wearer during use. The fastening tab is preferably strip-shaped and comprises in CD a fastening portion through which it is attached to the rear portion of the diaper or the side panel, a gripping portion opposite to said fastening portion which is gripped by the user when extending the fastening tab and applying the diaper to the wearer, and an intermediate portion arranged between the fastening portion and the gripping portion. While the term "fastening portion" which relates to that portion of the fastening tab which is secured to the absorbent article, for example, through an adhesive bonding strip and/or other bonding means, is defined by its construction the term "gripping portion" is functionally defined as that portion of the fastening tab which is gripped by a user when applying the absorbent article to a wearer. Thus, depending, for example, on the construction of the absorbent article or the side panel, respectively, the gripping portion may in some cases be provided by the fingerlift, in other cases by the portion of the fastening tab protruding beyond the distal longitudinal edge of the absorbent article or the side panel, respectively, and yet in other cases by the portion of the fastening tab exposed through the cut-out portion, optionally together with the portion of the fastening tab protruding beyond the distal longitudinal edge of the absorbent article or the side panel, respectively. The intermediate portion of the fastening tab is in each case the portion bridging the distance between the fastening portion and the gripping portion, respectively.

The fastening tab used in the present invention is elastically extendable or stretch-activatable to become elastically extendable, respectively. Elastically extendable means that the fastening tab will substantially resume its original shape after being stretched to moderate extensions of between 50 - 200 % so that the difference of the length of the fastening tab in CD in the non-extended state after extension and relaxation relative to the original state prior to relaxation preferably is less than 20% and more preferably less than 10%. The term "original state" refers to the relaxed state of the fastening tab prior to the corresponding relaxed state after the (n+1)-th extension cycle with n=0, 1, 2, ..... That means that in case n = 0, comparison is made between the length in the relaxed state after the 1^{st} extension relative to the length in the virgin state of the fastening tab prior to any stretching and so on; at a given time, comparison is made between the length of the fastening tab in the relaxed state at such point in time with the length obtained in the relaxed state subsequent to one stretching and relaxation cycle.

The fastening tab preferably comprises a backing which bears, is bonded to or integrally includes, respectively, functional components such as, for example, an elastic or stretch-extendable means and one or more mechanical fastening means such as one or more hook patches. The backing may comprise further functional components such as, for example, one or more non-woven layers to impart a cloth-like appearance to the fastening tab and or to improve its aesthetical appearance, respectively and/or at least one finger-lift portion representing or forming part of the gripping portion as the case may be.

The backing preferably includes an elastically extendable film or laminate which may be selected from a broad range of films and laminates including single- or multilayered films, coextruded films, laterally laminated films or films comprising foam layers. The elastic elastically extendable films or laminates preferably comprise one or more elastic materials which are selected from a group A-B or A-B-A block copolymers such as styrene/isoprene, butadiene or ethylene-butylene/styrene block copolymers, elastomeric polyurethanes, elastic ethylene copolymers such as ethylene-vinyl acetastes, ethylene/propylene copolymer elastomers or ethylene/propylene/diene terpolymer elastomers, elastomeric polyamide materials and elastomeric polyester materials. Elastomeric materials which are suitable in the present invention will sustain only small permanent set following deformation and relaxation which set is preferably less than 20% and more preferably less than 10% of the original length at elongations of e.g. 100 - 200 %.

The elastically extendable film or laminate useful in the present invention may be elastic in the virgin state and consequently not require an activation step. A preferred example of such embodiment is a single layer elastic film comprising one or more of the elastomeric materials specified above. The thickness of such single layer elastic film preferably is between 25 µm and 150 µm and more preferably between 50 µm and 100 µm.

Suitable elastically extendable films or laminates which do not require an activation are commercially available from Epurex or Clopay companies.

The elastically extendable films or laminates useful in the present invention may not be elastic in the virgin state but may require an activation step in order to render them elastic. A preferred example of such an embodiment is a multi-layer film comprising at least one non-elastomeric (or less elastomeric relative to the core layer, respectively) skin layer and at least one elastomeric core layer which layers are preferably co-extensive along the extension of the fastening tab. US 5,501,679 discloses activated micro-textured elastomeric laminates comprising at least one elastomeric core layer and at least one thin skin layer. The activated micro-structured elastomeric laminate is preferably prepared by co-extrusion of the core and skin layer followed by stretching the laminate past the elastic limit of the skin layers and then allowing the laminate to recover. It is disclosed in US '679 that the shrink recovery mechanism of the laminate can be influenced by varying the thickness of the skin layer or layers. It is furthermore disclosed in US '679 that the texture of the micro-structured laminate can be influenced by varying the manner in which the film is stretched past its elastic limit. It is disclosed that the laminate can be stretched uniaxially, sequentially biaxially or simultaneously biaxially.

Activatable composite laminate webs having an elastic core layer and at least one skin layer which is less elastic than the core layer are described, for example, in EP 0,521,883. EP '883 also discloses the corresponding activated elastic composite laminates. The core layers may be formed by the elastic materials disclosed above. The skin layer preferably is a polyolefin such as polyethylene, polypropylene, polybutylene or a polyethylene-polypropylene copolymer, but may also be wholly or partly polyamide such as nylon, polyester such as polyethylene terephthalate, polyvinylidene fluoride, polyacrylate such as poly(methyl methacrylate)(only in blends) and the like, and blends thereof. Further details on the construction of the activatable or activated laminate, respectively, can be taken from EP '883 B1, p. 4, In. 20 - p.5, In. 43 which passage is herewith incorporated by reference.

In the laminates of EP '883, the at least one skin layer and the at least one core layer form preferential activation regions and non-preferential activation regions wherein said at least one core layer is substantially elastomeric in at least said preferential activation regions. The at least one skin layer and/or the at least one core layer are provided such that when the multilayer laminate is stretched, said preferential regions can elongate and recover, in the elongated regions, to an elastic state. Nonwoven materials such as, for example, garments can be applied to the activatable or activated laminate, respectively, by ultrasonic welding, heat sealing and adhesives.

The laminates of EP '883 described above can be activated by a so-called post laminate-formation modulus or stress treatment in order to generate the required preferential activation regions and the non-preferential activation regions, respectively. Such treatment may include post formation annealing, selective crosslinking or selective plasticization. Post formation stress localization can be effected by localized corona treatment, mechanical ablation, scoring, cutting out laminate material, indentation or controlled localized stretching including the so-called "ring-rolling" process.

The controlled localized stretching treatment method is described to some detail in US 5,167,897. US '897 refers to a zero strain stretch laminate which comprises a first ply of a stretchable and elastomeric material and a second ply comprising an elongatable but not necessarily elastomeric material such as, for example, a nonwoven material. The two plies are attached to each other while in a substantially untensioned ("zero strain") condition which is also referred to above and below as "non-extended" or "relaxed". The zero strain stretch laminate is fed into a pair of opposed pressure applicators to effect incrementally stretching while subjecting opposed peripheral edge portions of said laminate web to restraint. The second ply will, upon stretching of the laminate, be at least to a degree permanently elongated so that, upon release of the applied tensile forces, it will not return to its original undistorted configuration. Depending on the properties of the second ply material and the degree of incremental stretching, the second ply material may undergo local Z-direction rupturing or Z-direction bulking, respectively, resulting in subsequent elastic extensibility in the direction of initially stretching.

Activatable laminates having at least one elastic core layer and at least one non-elastic or less elastic (relative to the core layer) skin layer are commercially available from 3M Co. under the designation UKME microelastic film.

US 6,159,584 discloses an extensible elastic tab comprising a coextruded elastic film with at least one elastic layer and at least one second layer on at least a first face of the elastic layer with at least one face of the coextruded elastic film attached to at least a partially extensible nonwoven layer. The partially expandable or extensible nonwoven layer has at least one first portion with limited extensibility in a first direction and at least one second inextensible portion in the first direction. The extensible elastic tab when stretched to the extension limit of the first portion or portions in the first direction will elastically recover at least 1.0 cm, preferably at least 2 cm providing an elastic tab having a Useful Stretch Ratio of at least 30 %. The Useful Stretch Ratio includes the portion of the elastic recovery length having an elastic recovery force of greater than 20 grams/cm force, but below a given extension which generally is 90 % of the extension limit. The extensible elastic tab of US '584 is suitable for being used in the absorbent articles or side panels, respectively, of the present invention.

The fastening tab suitable for use in the present invention preferably comprises an extendable film or laminate as a backing, i.e. the elastic film extends from the fastening portion to the gripping portion. It is, however, also possible that the fastening tab comprises a non-elastic backing which extends at least partly over the extension of the backing in CD to which an extendable film or laminate is attached. EP 1,133,967 discloses, for example, a fastening tab comprising an elastic laminate which is bonded to a first and a second inelastic backing section which are arranged opposite to each other. The elastic laminate comprises an extendable film or backing having on at least a first major side thereof an expandable fibrous layer of non-woven thermoplastic polymer fibres. EP 1,449,505 discloses a fastening tab comprising two backing portions adjacent to each other which essentially extend across the extension of the fastening tab in CD (i.e. from the fastening portion to the gripping portion). A blanking film and an extendable film or laminate are attached to the two backing portions in the area where these are adjacent to each other so that the laminate can be extended.

The fastening tab useful in the present invention comprises a mechanical fastening means which is mechanically engagable with a complementary fastening means and thus forms part of a mechanical closure system. A suitable mechanical closure system comprises two engagable fastening means, one of them being a hook (or male) fastener means and the other being a loop (or female) fastener means. The fastening means arranged on the fastening tab suitable in the present invention may comprise male (hook) fastener means or female (loop) fastener means, respectively. The fastening tab suitable in the present invention preferably comprises one or more male fastener means such as one or multiple strips or pieces. The male fastener means may comprise male fastener elements having any shape such as hooks, "T's", "mushrooms", "disks" or any other shape as are well known in the art. The hook fastener means preferably comprises a backing from which the individual fastening elements emanate. The hook fastener means may be manufactured from a wide range of materials including nylon, polyester, polyolefins or any combination of these. Preferred hook fastener means are commercially available from 3M Company, St. Paul, U. S. A.

The complimentary loop fastener means may be comprised of woven or non-woven fabrics or any other suitable material which can engage with the complementary hook fastener means. Suitable loop fastening means are commercially available, for example, from 3M Company, St. Paul, U. S. A.

In case the fastening tab suitable in the present invention comprises hook fastener means, it may be necessary and/or desirable to apply a patch of complementary loop material as a landing zone, for example, in the front portion (or waistband portion) of the absorbent article. In other cases such as, for example, where the outer surface of the back sheet of the absorbent article is formed by a non-woven material, the hook fastening means may be selected to interact directly with the outer surface of the back sheet so that a separate landing zone is not required (so-called hook-to-back sheet bonding).

The hook fastening means is typically provided as a hook patch comprising a backing from which the individual hook element emanate. The hook patch may be attached to the backing of the fastening tab by adhesive bonding, ultrasonic bonding, thermal bonding, fusing or any other attachment means.

If the fastening tab comprises an adhesive fastening means it may be necessary or desirable to include a separate landing zone for receiving an adhesive attachment of the fastening means thereon.

Fastening tabs useful in the present invention preferably comprise one or more mechanical fastening elements. Adhesive fastening means may be used in addition to one or mechanical fastening means to provide for an additional bonding mechanism. Such additional adhesive means preferably comprise a pressure-sensitive adhesive.

The fastening tab may comprise other components such as, for example, finger-lifts. The finger-lift is a gripping aid preferably attached at the edge of the fastening tab to facilitate gripping of the fastening tab by the user. The finger-lift may comprise, for example, a polymeric film which is attached to and folded around such edge of the backing in the gripping portion (whereby such fold in the finger-lift area is not considered as a fold of the fastening tab). The finger-lift may also be provided by attaching a small reinforcing polymeric film which may be, for example, 3-10 mm wide in CD, onto the end section of the gripping portion so that it is essentially flush with the edge of the backing in the gripping portion in MD. In another preferred embodiment, a hook fastening means may be included into the fastening tab so that it extends essentially to the edge of the backing in the gripping portion; a finger-lift can then be provided by crushing the hook elements of the fastening portion in a small area which can be, for example, 3-10 mm wide in CD, adjacent to such edge of the baking in the gripping portion.

The fastening tab useful in the present invention is essentially flat at least in the non-extended state which means that the fastening tab is fold-less. This means that the fastening tab is neither folded onto itself, i.e., it does, for example, not exhibit a so-called Z-fold arrangement nor is the fastening tab or parts thereof, respectively, folded around, for example, the longitudinal edge of the diaper or the distal longitudinal edge of the side panel, respectively. Thus, no folding operation is required when applying the fastening tab useful in the present invention to the absorbent article or the side panel, respectively, which facilitates and/or speeds up the manufacturing process. Also, folded or z-fold arrangements usually add some thickness to the absorbent article or the side panel, respectively, which may result in an undesirable stiffness of parts of the absorbent article or side panel, respectively.

The fastening tab of the present invention may exhibit various shapes and geometries. The fastening tab can exhibit, for example, an essentially rectangular shape with the long side preferably extending in CD. The gripping portion and, optionally, the intermediate portion of the fastening tap may also exhibit, for example, a generally tapered shape towards the end of the gripping portion. Other shapes of the fastening tab such as, for example, a triangular shape, a rhomboidic shape or the like are also possible. It is also preferred that one or more of the edges of the fastening tab in MD and/or CD exhibit, exhibit a curvature, a wavy shape or the like.

The dimensions of the fastening tab useful in the present invention can vary broadly. The extension of the fastening tab in the CD (i.e. from the edge of the fastening portion to the edge at the gripping portion) may vary, for example, between 45 mm and 90 mm and more preferably between 50 and 80 mm. The extension of the fastening tab in MD may vary between 15 and 60 mm and more preferably between 20 and 45 mm. These dimensions refer in cases of a non-rectangular shape of the fastening tab to the longest extensions in CD and MD, respectively.

The fastening tab is secured to the absorbent article or the side panel, respectively, through its fastening portion whereby such attachment can be effected by various bonding means including, for example, adhesive bonding, mechanical bonding, thermal bonding, welding, ultrasonic bonding or any combination thereof. Suitable adhesives include, for example, pressure sensitive adhesives or hot-melt adhesives. Mechanical means include, for example, stitching, attachment via mechanical fastening system and the like. Thermal bonding, welding and/or ultrasonic bonding typically result in fusing together the bottom surface of the fastening portion and the exposed surface of the absorbent article or the side panel, respectively, at the interface between such surfaces. A combination of adhesive bonding and ultrasonic bonding is preferred. The bonding between the fastening portion and the absorbent article or the side panel, respectively, needs to be sufficiently strong so that it does not break when the fastening tab is extended and when the diaper is attached to the wearer.

The fastening portion of the fastening tab usually has an extension of between 5 and 30 mm in CD and more preferably between 10 and 25 mm in CD. The ratio of the extension of the fastening portion in CD over the extension of the fastening tab in CD preferably is between 0.15 and 0.4 and more preferably between 0.15 and 0.3. The fastening portion preferably extends in MD essentially over the full width of the fastening tab. The dimensions given refer in cases of a non-rectangular shape of the fastening portion to its longest extensions in CD and MD, respectively.

The gripping portion is arranged at the end of the fastening tab opposite to the fastening portion. The gripping portion represents the portion of the fastening tab in the non-extended state which is gripped by the user when applying the absorbent article to the wearer. The gripping portion preferably comprises a finger-lift, if present, but it may also comprise part or all of the one or more mechanically fastening means which are usually arranged in some distance from the edge of the gripping portion. The extension of the gripping portion also depends on the design of the fastening tab and its arrangement on the absorbent article or the side panel, respectively. If the fastening tab is arranged, for example, in a single sided configuration so that the edge of the gripping portion in MD is adjacent to the longitudinal edge of the absorbent article or the distal longitudinal edge of the side panel, respectively, and if the absorbent article or the side panel comprises a cut-out section next to the longitudinal edge of the absorbent article or the distal longitudinal edge of the side panel, respectively, in order to facilitate the gripping of the fastening tab, the portion of the fastening tab exposed through the cut-out section can be considered as a gripping portion. In other constructions wherein the edge of the gripping portion in MD distally projects beyond the longitudinal edge of the absorbent article or the side panel, respectively, the projecting portion of the fastening tab may be considered as a gripping portion.

The gripping portion of the fastening tab usually has an extension of between 3 and 30 mm in CD and more preferably of between 5 and 25 mm in CD. The ratio of the extension of the gripping portion in CD over the extension of the fastening tab in CD preferably is between 0.05 and 0.3 and more preferably between 0.10 and 0.25. The dimensions given refer in cases of a non-rectangular shape of the gripping portion to its longest extensions in CD and MD, respectively.

In the present invention the fastening tab is attached to the absorbent article or the side panel, respectively, so that less than 25 %, more preferably not more than 20 %, especially preferably not more than 15% and especially preferably not more than 10% of its extension in CD in its non-extended state protrudes beyond or undershoots, respectively, the longitudinal edge of the diaper. The length of the fastening tab in CD is measured in cases of a non-rectangular shape of the fastening tab as its longest extensions in CD. Likewise, the distance between the distal edge of the fastening tab and the longitudinal edge of the diaper is measured in cases of a non-rectangular shape of the protruding or undershooting portion as its longest extensions in CD. For such measurements the directions of the longest extension of the fastening tab and of the protruding portion in CD, respectively, do not need to match, i.e. these directions are measured independently from each other. The above percentages are absolute values what means that the fastening tab may protrude beyond or undershoot, respectively, the longitudinal edge of the absorbent article or the distal longitudinal edge of the side panel, respectively, by such percentages.

In an alternative arrangement of the present invention the fastening tab is attached to the absorbent article or the side panel, respectively, so that the absolute value of the distance between the distal edge of the fastening tab and the longitudinal edge of the absorbent article or the distal longitudinal edge of the side panel, respectively, is less than 15 mm, more preferably not more than 12.5 mm and especially preferably not more than 10 mm. The distance between the distal edge of the fastening tab and the longitudinal edge of the absorbent article or the distal longitudinal edge of the side panel, respectively, is measured in cases of a non-rectangular shape of the protruding or undershooting portion at its longest extensions in CD. The above distance is an absolute value what means that the fastening tab may protrude beyond or undershoot, respectively, the longitudinal edge of the absorbent article or the distal longitudinal edge of the side panel, respectively, by such distance.

Preferably, the fastening tab is arranged on the absorbent article or the side panel, respectively, so that it protrudes the longitudinal edge of the diaper or the distal longitudinal edge of the side panel, respectively, rather than undershooting such edges. This arrangement facilitates gripping of the fastening tab by the user and reduces the elastic extension to be obtained when securing the absorbent article to a wearer.

The fastening tab is preferably arranged on the absorbent article or the side panel, respectively, so that the direction of its maximum extension in CD forms an angle of between ± 60°, more preferably between ±30 ° and especially preferably essentially normal relative to the MD direction of the absorbent article.

In a preferred embodiment, the fastening tab is preferably arranged in a single-sided configuration on the absorbent article or the side panel, respectively. The term "single-sided configuration" as used above and below means that the fastening tab is supported in its non-extended state essentially along its extension in CD by the back sheet or the top sheet, respectively, of the absorbent article or the side panel. The term "essentially along its extension in the CD" means that the fastening tab is extending in its non-extended state beyond a longitudinal edge of the diaper or the side panel, respectively, to an extent so that there is no danger for the fastening tabs to be ripped off during the manufacturing process. Preferably, the fastening tab protrudes beyond or undershoots the longitudinal edge of the diaper or the distal longitudinal edge of the side panel, respectively, within the limits as indicated above. In the single side configuration the fastening tab is fold-less in its non-extended state, i.e. it is neither folded upon itself nor folded around a longitudinal edge of the absorbent article or side panel, respectively.

In the single-side configuration, the fastening tab may be attached to the absorbent article or the side panel, respectively, so that its gripping portion is arranged distally relative to a center line of the absorbent article or the side panel, respectively, in MD direction whereas the fastening portion is arranged proximally relative to such center line. In such arrangement, the fastening tab is preferably arranged on the back sheet of the absorbent article as is shown, for example, in Fig.'s 1a and 1b and 3a or 3b below. If the fastening tab is attached in such arrangement, for example, to the rear waist portion of a diaper (see, for example, Fig.'s 7a and 7b) the gripping portion can be conveniently gripped by the user and pulled beyond a longitudinal edge of the absorbent article or side panel, respectively, and attached to the front waist portion thereby attaching the absorbent article to a wearer. If one or more mechanical fastening means are used these are facing and thus may interact with the exposed surface of the back sheet. If the exposed surface of the back sheet is formed, for example, by a non-woven layer such interaction may provide a holding down effect which eliminates any flagging and is advantageous during manufacturing and storing of the absorbent article or the side panel, respectively. The fastening tab may also be attached to the top sheet of the absorbent article or the side panel, respectively, which is exemplified, for example, in Fig.'s 5a and 5b. In such arrangement the mechanical fastening element is facing the wearer's skin so that such arrangement is usually less preferred.

In the single-sided configuration, the fastening tab may alternatively be attached to the absorbent article or the side panel, respectively, so that its fastening portion is arranged distally relative to a center line of the absorbent article or the side panel in MD, respectively, whereas the gripping portion is arranged proximally relative to such center line. When securing the diaper to a wearer, the user will grip and lift the proximally arranged gripping portion. The fastening portion is folded around typically in the fastening portion and pulled beyond the longitudinal edge as is illustrated, for example, in Fig.'s 4b and 6b. If the fastening tab is attached to the back sheet of the absorbent article or the side panel, respectively, the fastening means of the fastening tab are facing away from the back sheet in the non-extended state of the fastening tab as is illustrated, for example, in Fig.'s 4a and 4b. If the fastening tab is attached to the top sheet the fastening means are facing the top sheet in the non-extended state of the fastening tab what is illustrated, for example, in Fig.'s 6a and 6b. Attaching the fastening tab in this single-side configuration to the top sheet is preferred since this orientation may provide a holding-down force as was explained above.

Single-sided configurations where the gripping portion is arranged distally from the MD center line and thus close to the longitudinal edge of the absorbent article or the distal longitudinal edge of the side panel, respectively, are preferred. The gripping portion and/or part or all of the mechanical fastening element, respectively, may extend beyond the longitudinal edge in order to facilitate gripping of the gripping portion by the user provided, whereby it is usually desirable that at least 75 %, more preferably at least 80 %, particularly preferably at least 85 %, especially preferably at least 90 % and most preferably at least 95 % of the longitudinal extension of the fastening tab in its non-extended state in CD are supported by the back sheet or the top sheet of the absorbent article or the side panel. The fastening tab may also be arranged so that it is fully supported in its non-extended state in CD by the back sheet or the top sheet of the absorbent article or the side panel, respectively. The absolute value of the distance between the distal end of the gripping portion (irrespective of whether it is arranged distally or proximally from the longitudinal edge) and such longitudinal edge preferably is between 0 - 20 mm, more preferably between 0 - 10 mm and most preferably between 0 - 5 mm.

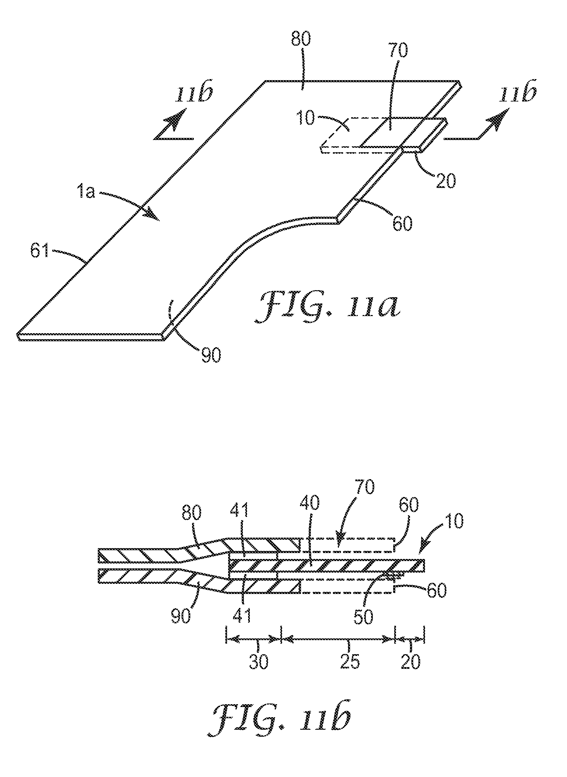

In another preferred embodiment the absorbent article or the side panel, respectively, exhibit a distally open cut-out area next to the longitudinal edge of the absorbent article or the side panel, respectively, through which part of the fastening tab is exposed. The cut-out portion may be shaped irregularly or regularly; it may exhibit, for example, a curved shape as is exemplified in Fig.'s 3a and 3c or, for example, a polygonic shape such as rectangular or quadratic (see Fig. 11a). The cut-out portion may be applied to either of the back sheet or the top sheet or to both of them whereby in the latter case the shape and geometry of the cut-out areas applied to the back sheet and the top sheet, respectively, may be selected independently from each other. Applying identical cut-out areas to both the top sheet and the back sheet is preferred. The extension of the cut-out area in CD is measured as the maximum distance between the extrapolated longitudinal edge line of the absorbent article or the distal longitudinal edge line of the side panel, respectively, and the proximal border (cut line) of the cut-out area. The extension of the cut-out area preferably is between 0 - 20 mm, more preferably between 0 - 10 mm and most preferably between 0 - 5 mm. In a preferred embodiment, the cut-out area exposes only part of the mechanical fastening element so that the mechanical fastening element when arranged in a face-down configuration where the mechanical fastening elements faces the back sheet or top sheet, respectively, may still engage with the back or top sheet of the absorbent article or side panel which preferably comprises a non-woven material which can interact with the mechanical fastening element to provide for some peel and/or shear interaction. This is beneficial in order to maintain some holddown effect even when a cut-out area is present.

In another preferred embodiment, the fastening tab is preferably arranged in a sandwich configuration between the top sheet and the back sheet of the absorbent article or the side panel, respectively. The term "sandwich configuration" as used above and below means that the fastening tab is supported in its non-extended state essentially along its extension in CD between the back sheet and the top sheet, respectively, of the absorbent article or the side panel. The term "essentially along its extension in the CD" means that the fastening tab is only extending in its non-extended state beyond a longitudinal edge of the diaper or the side panel, respectively, to an extent so that there is no danger for the fastening tabs to be ripped off during the manufacturing process. If no cut-out area is applied, the fastening tab preferably protrudes beyond the longitudinal edge of the diaper or the distal longitudinal edge of the side panel, respectively, so that gripping of the fastening tab by the user is facilitated. If a cut-out area is applied the fastening tab may also preferably be arranged so that it undershoots the longitudinal edge of the diaper or the distal longitudinal edge of the side panel, respectively. In the sandwich configuration the fastening tab is fold-less in its non-extended state, i.e. it is neither folded upon itself nor folded around a longitudinal edge of the absorbent article or side panel, respectively.

In the sandwich configuration the back sheet may be attached to the top sheet adjacent to the fastening tab thereby creating a pocket further supporting the fastening tab. Such bonding may be affected, for example, by applying ultra-sonic bonding points around and adjacent to the longitudinal CD edges of the fastening tab. When applying such pocket structure care should be taken, however, that the extension of the fastening tab when pulling it, is not adversely affected and that, in particular, the fastening tab, the back sheet and/or the top sheet are not damaged by the stretching and relaxing operations.

If the inner surfaces of the back sheet and/or the top sheet facing the fastening tab are capable to interact with a mechanical fastening means of such fastening tab and if the absorbent article or the side panel, respectively, do not comprise a cut-out area it is usually preferred that part of the mechanical fastening means protrudes beyond the longitudinal edge of the absorbent article or the distal longitudinal edge of the side panel, respectively. This allows to fine-tune the interaction between the fastening means and the respective surface of the top sheet or the back sheet, respectively, so that such surface is not damaged by the shear force which may arise when pulling out the fastening tab. The interaction between the one or more mechanical fastening means and the inner surface of the top sheet or the back sheet, respectively, may further be optimized by applying an appropriately shaped cut-out area.

Cut-out areas are furthermore preferred in the absorbent articles or side panels of the present invention, respectively, because they facilitate gripping of the fastening tab by the user and because they may be aesthetically pleasing.

The present invention also relates to a method of manufacturing an absorbent article or a side panel for an absorbent article, respectively. While certain variations of such methods are provided within the present inventions, all such methods include a step comprising the attachment of fastening tabs to absorbent articles or side panels or to a web of absorbent articles or side panels, respectively. The method differs from conventional manufacturing methods in that the fastening tabs are applied to such web or the absorbent articles or side panels obtained from such web, respectively, in a single-sided configuration as was described above. This arrangement does not require folding over the fastening tabs in their non-extended state in order to avoid excessive flagging and/or prevent the fastening tabs from being ripped off during manufacturing as in conventional processes which may result in an interruption of the manufacturing process. This allows to run the manufacturing line at a higher line speed as compared to a conventional method which may provide a cost-benefit. The folding step is a relatively complicated operation which requires additional equipment in the diaper line.

Especially preferred are essentially waste-less manufacturing methods which preferably includes processing a web of side panels into an even number of sub-webs (i.e. 2n sub-webs with n = 1, 2, 3 and so) whereby a pair of complimentary side panels can be obtained from a pair of adjacent sub-webs.

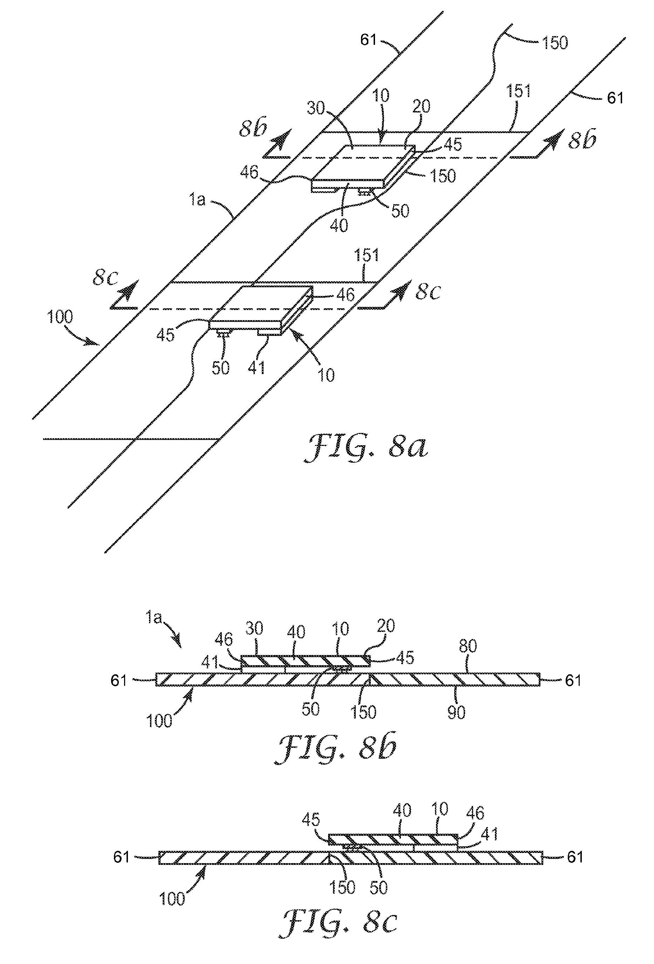

This is exemplified in Fig. 8a where a sequence of fastening tabs is arranged along a cut line regularly meandering along the center line of the web and extending in MD. Subsequent fastening tabs are attached so that they are arranged on the precursor of the corresponding side panel in a single-side configuration with the gripping portion facing the cut line. This requires in the exemplary embodiment of Fig. 8a that subsequent fastening tabs are rotated relative to each other by an angle of about 180°. Individual pairs of left and right side panels are obtained by applying the MD cut line and a sequence of cut lines in CD.

Further examples of such essentially waste-free methods are shown in Fig.'s 9 a, 9b and Fig. 10a, respectively. The method of Fig. 10a is also referred as waste-less although the cut-out areas are discarded. Fig.'s 9a, 9b and 10a exemplify how the pair of complimentary side panels may be attached, for example, to an essentially rectangular chassis of an absorbent article. This may require that the side panels are crossed over (as in Fig.'s 9a and 10a), rotated or moved around otherwise to bring them into the desired orientation relative to the absorbent article such as, for example, the diaper chassis.

If desired, the webs or sub-webs of side panels may be wound up in the form of rolls which may be planetary or level-wound. The rolls may then be unwound at the manufacturing line of the absorbent articles so that a sequence of side panels can be conveniently obtained. Separating the process of manufacturing a web or sub-webs, respectively, of side panels from the process of manufacturing a web of absorbent articles may simplify the manufacturing process and allow for less complicated, more robust manufacturing.

Alternatively, the process of manufacturing a web or sub-webs, respectively, of side panels and the process of manufacturing a web of absorbent articles may be integrated into one production process and the corresponding production line so that the side panels are manufactured and supplied "in situ".

### Detailed description of the drawings

Fig. 1a shows an embodiment of a side panel 1a of the present invention bearing on its back sheet 80 a fastening tab 10 in its non-extended state. The side panel 1a has a proximal longitudinal edge 61 (which will be attached to the chassis 1b of an absorbent article 1, not shown) and a distal longitudinal edge 60. The fastening tab 10 comprises a backing 40 extending across the fastening portion 30, the intermediate portion 25 and the gripping portion 20 of the fastening tab 10. The fastening tab 10 is positioned on the side panel 1a so that its gripping portion 20 is adjacent to the distal longitudinal edge 60 of the side panel 1a whereas the fastening portion 30 of the fastening tab 10 is arranged proximally to such distal longitudinal edge 60. In the embodiment of Fig. 1a the distal edge 45 of the fastening tab 10 in its non-extended state is essentially aligned with the longitudinal edge 60 of the side panel 1a beneath, that means that the distal edge 45 of the fastening tab 10 does essentially neither distally protrude nor proximally undershoot the longitudinal edge 60 of the side panel 1a (see also the cross-sectional view of Fig. 1b). The fastening portion 30 of the fastening tab 10 is attached to the back sheet 80 of the side panel via the adhesive strip 41. The fastening portion 30 is furthermore secured to the backing 80 of the side panel by ultrasonic bonding points 42. The fastening tab 10 furthermore comprises one or more mechanical fastening means 50 such as, for example, one or multiple strips or pieces each having male fastening elements such as hooks arranged on and emanating from a continuous back strip. The male fastening elements are capable of engaging with a complementary fastening means such as, for example, a non-woven which may be provided by a non-woven back sheet of the absorbent article or a landing zone in the front waist region of the absorbent article (not shown). The male fastening elements are facing the back sheet 80 of the side panel 1a so that the male fastening elements of the mechanical fastening element 50 may engage with the back sheet 80 of side panel 1a. This interaction may hold the fastening tab 10 down on the back sheet 80 during manufacturing or storing thus preventing the fastening tab 10 from popping up or flagging. The back strip of the mechanical fastening means 50 may be attached to the backing 40 of the fastening tab 10 by an adhesive layer (not shown) or it may be bonded thermally, e.g. fused, to the backing 40.

Fig. 1b shows a cross sectional view of the side panel 1a of Fig. 1a across the line 1b-1b indicated therein. The backing 40 of the fastening tab extends from its proximal edge 46 to its distal edge 45 and comprises the fastening portion 30, the intermediate portion 25 and the gripping portion 20. The distal edge 45 of the fastening tab 10 is essentially flush with the distal longitudinal edge 60 of the side panel 1a. The fastening tab 10 of this embodiment is typically gripped at its fingerlift which is represented by the distance between the distal edge 45 of the fastening tab 10 and the distal edge of the fastening means 50. The mechanical fastening means 50 is thus considered in this embodiment as part of the intermediate portion 25. The mechanical hook elements of the fastening means 50 are facing the back sheet 80 of the side panel 1a. The fastening tab 10 is adhered to the back sheet 80 of side panel 1a in the fastening zone via adhesive layer 41 and ultrasonic bonding points 42 (not shown in Fig. 1b).

Fig. 2a shows the side panel 1a of the embodiment of Fig. 1a wherein the fastening tab 10 is in an extended state. Due to the application of a force of the user in CD the fastening tab is elongated and is protruding with its gripping portion 20 and part of its intermediate portion 25 which includes the fastening means 50, beyond the longitudinal edge 60 of the side panel 1a. The fastening means 50 is ready to be engaged via its mechanical hook elements to a respective complementary fastening element on the absorbent article (not shown) in order to secure the absorbent article 1 to the wearer.

Fig.2b is a cross-sectional view of the side panel 1a of Fig. 2a along the line 2b-2b indicated in Fig. 2a.

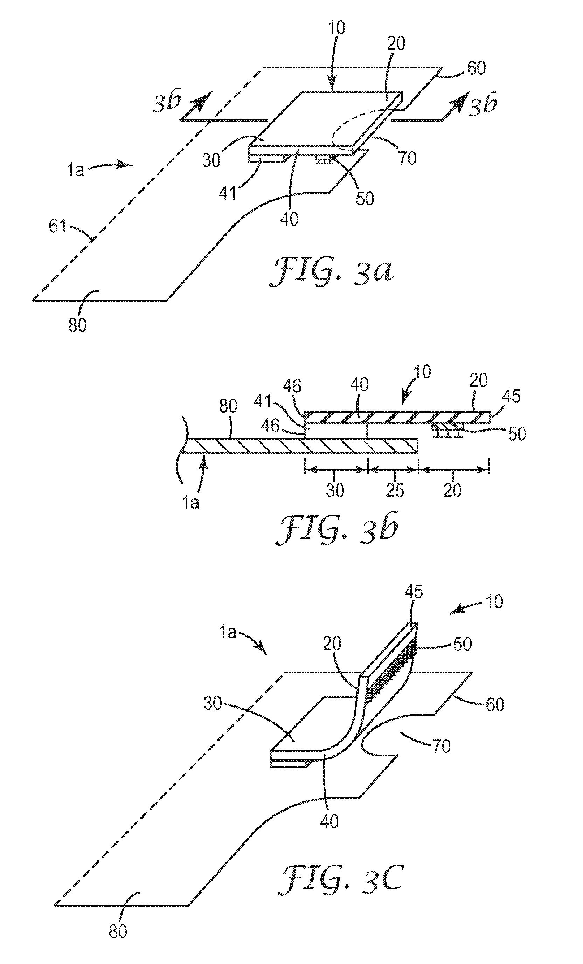

Figs. 3a shows another embodiment of a side panel 1a of the present invention bearing on its back sheet 80 a fastening tab 10 in its non-extended state. The side panel 1a has a proximal longitudinal edge 61 (which will be attached to the chassis 1b of an absorbent article 1, not shown) and a distal longitudinal edge 60. The fastening tab comprises a backing 40 extending across the fastening portion 30, the intermediate portion 25 and the gripping portion 20 of the fastening tab 10. The fastening tab 10 is positioned on the side panel 1a so that its gripping portion 20 is adjacent to the distal longitudinal edge 60 of the side panel 1a whereas the fastening portion 30 of the side panel 1a is arranged proximally to such distal longitudinal edge 60. The side panel 1a exhibits a cut-out area 70 through which the gripping portion 20 is exposed; it can be more clearly seen from the cross-sectional view of Fig. 3b which extends along the line 3b-3b shown in Fig. 3a, that the gripping portion 20 comprises in the embodiment of Fig.'s 3a - 3c both the fingerlift and the mechanical fastening means 50 comprising mechanical hook elements. The cut out portion extends proximally from the longitudinal edge 60 beneath the extension of the fastening tab 10 in MD. The extension of the fastening tab 10 in MD exceeds the extension of the cut-out area 70 in MD so that the fastening tab 10 covers the cut out area 70. Thus the fastening tab 10 is also supported by the back sheet 80 of the side panel 1a in the area of the cut-out 70. The cut-out area has a semi-circular or semi-ellipsoidal shape. Gripping of the gripping portion 20 of the fastening tab 10 is facilitated through the cut-out area 70. In the embodiment of Fig. 3a the distal edge 45 of the fastening tab 10 in its non-extended state is essentially aligned with the extrapolation of the longitudinal edge 60 of the side panel 1a through the cut-out area 70, that means that the distal edge 45 of the fastening tab 10 does essentially neither distally protrude nor proximally undershoot the longitudinal edge 60 of the side panel 1a.The fastening portion 30 of the fastening tab 10 is attached to the back sheet 80 of the side panel via the adhesive strip 41. As can be seen in Fig. 3a, the extension of the mechanical fastening element 50 in MD is larger than the corresponding MD extension of the cut-out area 70. Thus the male fastening elements (hooks) of the mechanical fastening element 50 may still engage with the back sheet 80 of the absorbent article of side panel 1a and provide some hold down effect in spite of the presence of the cut-out area.

Fig.3b is a cross-sectional view of the side panel 1a of Fig. 3a in its non-extended state along the line 3b-3b indicated in Fig. 3a.

Fig. 3c is a perspective view of the side panel 1a of Fig.'s 3a and 3b showing the fastening tab 10 in a lifted condition which may occur initially when the gripping portion 20 is gripped by a user. This view better displays the cut-out area 70.

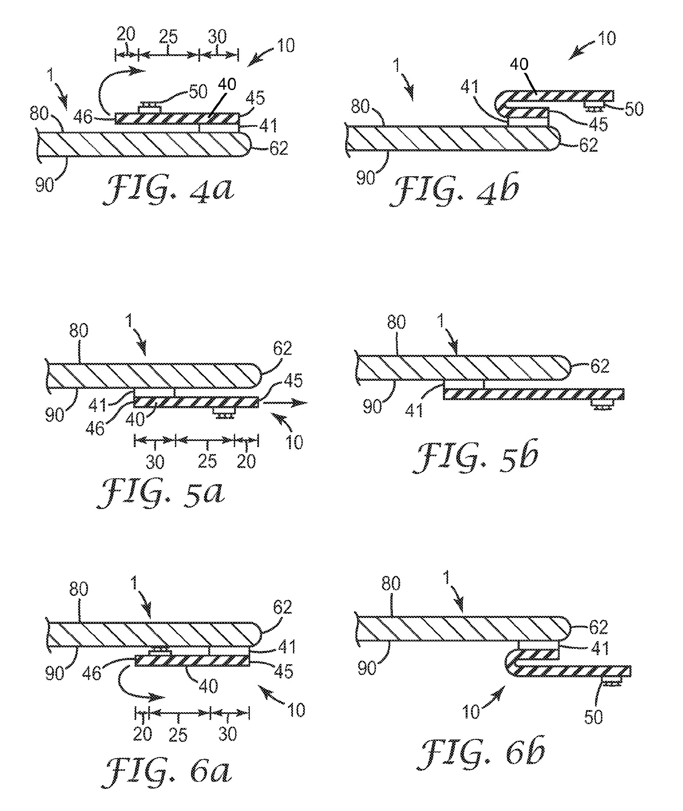

Figs. 4 - 6 show schematic cross-sectional partial views of various embodiments of absorbent articles 1 of the present invention.

Fig. 4a shows an embodiment of an absorbent article 1 of the present invention bearing on its back sheet 80 a fastening tab 10 in its non-extended state. The absorbent article 1 has longitudinal edges 62. The fastening tab 10 extends from its proximal edge 46 to its distal edge 45. The fastening tab 10 comprises a backing 40 extending across the fastening portion 30, the intermediate portion 25 and the gripping portion 20 of the fastening tab 10. The fastening tab 10 is arranged on the back sheet 80 of the absorbent article 1 so that its fastening portion 30 is facing the longitudinal edge 62 of the absorbent article 1. The distance between the longitudinal edge 62 of the absorbent article 1 and the distal edge 45 of the fastening tab is preferably small since such distance needs to be bridged by the fastening tab when it is extended so that the absorbent article can be applied to a wearer's body. The fastening tab 10 is secured to the back sheet 80 of the absorbent article 1 by means of an adhesive layer 41 arranged between the backing 40 of the fastening tab 10 and the exposed surface of the backing. The gripping portion 20 of the fastening tab 10 is arranged proximally relative to the fastening portion 30. In Fig. 4a the gripping portion 20 essentially corresponds to the fingerlift of the fastening tab 10 although the user may also grip the mechanical fastening means 50, i.e. part of the intermediate portion 25. The mechanical fastening element 50 is arranged on the side of the backing 40 of the fastening tab 10 opposite to the side of the backing 40 to which adhesive layer 41 is applied.

Upon use, as shown in fig. 4b, the fastening tab 10 is gripped and lifted by the user whereby the fasting tab 10 is being folded upon itself in the intermediate portion 25 close to the fastening portion 30. The user applies a pulling force typically in CD thereby elongating the fastening tab 10 beyond the longitudinal edge 62 of the absorbent article 1.

Fig. 5a shows another embodiment of an absorbent article 1 of the present invention wherein the fastening tab 10 is attached to the top sheet 90 of the absorbent article 1 rather than to its back sheet 80. The absorbent article 1 has longitudinal edges 62. The fastening tab 10 which is shown in Fig. 5a in its non-extended state, comprises a backing 40 extending across the fastening portion 30, the intermediate portion 25 and the gripping portion 20 of the fastening tab 10. The fastening tab 10 is positioned on the top sheet of the absorbent article 1 so that its gripping portion 20 is adjacent to the longitudinal edge 62 of the absorbent article 1. The fastening portion 30 of the absorbent article 1 is arranged proximally to such longitudinal edge 62. In the embodiment of Fig. 5a the distal edge 45 of the fastening tab 10 in its non-extended state is essentially aligned with the longitudinal edge 62 of the absorbent article 1. The fastening portion 30 of the fastening tab 10 is attached to the top sheet 90 of the absorbent article 1 via the adhesive strip 41. The gripping portion 20 of the fastening tab 10 is arranged proximally relative to the fastening portion 30. In Fig. 5a the gripping portion 20 essentially corresponds to the fingerlift of the fastening tab 10 although the user may also grip the mechanical fastening means 50, i.e. part of the intermediate portion 25. The mechanical fastening element 50 is arranged on the side of the backing 40 of the fastening tab 10 opposite to the side of the backing 40 to which adhesive layer 41 is applied.

Upon use, as shown in Fig. 5b, the fastening tab 10 is gripped and lifted by the user, who applies a pulling force typically in CD in order thereby extending the fastening tab 10 beyond the longitudinal edge 62 of the absorbent article 1.

Fig. 6a shows an embodiment of an absorbent article 1 of the present invention bearing on its top sheet 90 a fastening tab 10 in its non-extended state. The absorbent article 1 has a distal longitudinal edge 62. The fastening tab 10 extends from its proximal edge 46 to its distal edge 45. The fastening tab 10 comprises a backing 40 extending across the fastening portion 30, the intermediate portion 25 and the gripping portion 20 of the fastening tab 10. The fastening tab 10 is arranged on the top sheet 90 of the absorbent article 1 so that its fastening portion 30 is facing the longitudinal edge 62 of the absorbent 1. The distance between the longitudinal edge 62 of the absorbent article 1 and the distal edge 45 of the fastening tab is small since such distance needs to be bridged by the fastening tab when it is extended so that the absorbent article can be applied to a wearer's body. The fastening tab 10 is secured to the top sheet 90 of the absorbent article 1 by means of an adhesive layer 41 arranged between the backing 40 of the fastening tab 10 and the exposed surface of the top sheet 90. The gripping portion 20 of the fastening tab 10 is arranged proximally relative to the fastening portion 30. In Fig. 6a the gripping portion 20 essentially corresponds to the fingerlift of the fastening tab 10 although the user may also grip the mechanical fastening means 50, i.e. part of the intermediate portion 25. The mechanical fastening element 50 is facing the top sheet 90, i.e. it is arranged on the side of the backing 40 of the fastening tab 10 bearing adhesive layer 41.

Upon use, as shown in Fig. 6b, the fastening tab 10 is gripped and lifted by the user whereby the fasting tab 10 is being folded upon itself in the intermediate portion 25 close to the fastening portion 30. The user applies a pulling force typically in CD thereby elongating the fastening tab 10 beyond the longitudinal edge 62 of the absorbent article 1.

Fig.s 7a - 7c show disposable diapers representing absorbent articles according to the present invention.

Fig. 7a shows a diaper 1 having an essentially hourglass shape in an unfolded state. The diaper has a rear portion 2, a crotch portion 3 and a front portion 4. The diaper has a longitudinal edge 62 on each side. Two fastening portions 10 are attached to the back sheet 80 of the rear portion 2 of the diaper 1 in a single-side configuration whereby in the non-extended state of the fastening tabs 10 shown in Fig. 7a, the predominant portion of the fastening tab is supported by the back sheet 80 of the diaper 1. Only the gripping portion 20 each of the fastening tabs 10 is protruding beyond the respective longitudinal edge 62 of the diaper 1; such gripping portion of the fastening tab 10 is, however, so small that it does not result in an extent of flagging which is non-acceptable and/or it does not pose the risk of the fastening tabs 10 been ripped of or otherwise destroyed during the manufacturing of the diaper.

Fig. 7b is a schematic perspective view showing the diaper 1 of Fig. 7a being applied to a wearer. The diaper has been applied so that the top sheet 90 of the rear portion 2 is facing the back of the wearer whereas the top sheet of the front portion faces the waist of the wearer. The crotch area is forming openings for the legs. The rear portion of the diaper is folded upon the front portion of the diaper so that the fastening means 50 of the fastening tabs 10 which have been extended and pulled by the user beyond the longitudinal edges 62, are facing the back sheet 80 of the front portion 4. The male fastening elements (hooks) of the fastening means 50 are secured to the back sheet 80 of the front area 4; if desired a strip of a female fastening means (non-woven) may be applied to the back sheet 80 on the front portion 4 of the diaper 1 (not shown) to enhance the interaction between the male and female fastening means.

Fig. 7c shows another embodiment of a diaper 1 having an essentially rectangular chassis 1b bordered on each side by longitudinal edges 62. Two side panels 1a are attached to each side of the rear portion of the chassis 1b whereby the side panels 1a are attached via an area close to their respective proximal edges 61 to an area of the rear portion close to the longitudinal edges 62 of the diaper 1. Two fastening portions 10 are attached to the back sheet 80 of the side panels 1a each in a single-side configuration whereby in the non-extended state of the fastening tabs 10 shown in Fig. 7c, the predominant portion of the fastening tab is supported by the back sheet 80 of the side panel 1a. Only the gripping portion 20 each of the fastening tabs 10 is protruding beyond the respective longitudinal edge 60 of the side panel 1a; such gripping portion of the fastening tab 10 is, however, so small that it does not result in an extent of flagging which is non-acceptable and/or it does not pose the risk of the fastening tabs 10 been ripped of or otherwise destroyed during the manufacturing of the diaper.

Fig. 8a which illustrates a method of making side panels 1a of the present invention, shows a web 100 of side panels 1a from which complimentary pairs of side panels 1a according to the present invention can be cut. A sequence of fastening tabs 10 is attached to the web of side panels 1a so that the respective gripping portion 20 of such fastening tabs 10 is arranged adjacent to a (hypothetical) cut line 150 in MD at which a cut will be applied -together with cuts along cut lines 151 in CD - at a later stage of the process to provide a sequence of complementary (left and right) pairs of individual side panels 1a. The fastening tabs 10 are arranged on the web of side panels 1a that their respective distal edge is arranged adjacent to the cut line 150.

When the left and right pairs of side panels 1a are attached in the area of their proximal longitudinal edges 61 to the longitudinal edges of, for example a diaper 1 (not shown), the gripping portion 20 each of the fastening tabs 10 is arranged adjacent to the distal edges 60 of the side panels 1a; this means in other words that the fastening tabs are arranged in a single-side configuration on the side panels 1a.

The fastening tabs 10 are attached to the back sheet 80 of the side panels via the fastening portions 30 which each are facing away from the cut line 150; thus the fastening portions 30 are arranged proximally relative to the cut line 150 once the side panels have been attached to the chassis 1b of the absorbent article 1 (not shown). When being placed on the back sheet 80 of the web of side panels 1a the mechanical fastener means 50 of the fastening tabs 10 may engage with the back sheet 80 of the web of side panels 1a thereby holding the fastening tabs 10 in position relative to the (hypothetical) cut line 150. This is advantageous from a manufacturing point of view.

Fig.'s 8b and 8c provide cross-sectional views of the web of side panels along the lines 8b-8b and 8c-8c, respectively, shown in Fig. 8a. The single-sided arrangement of the fastening tabs 10 relative to the hypothetical cut line 150 can be seen. The distal edges 45 of the fastening tabs 10 are essentially flush with the (hypothetical) cut line 150.

Fig. 9a which illustrates another method of making side panels 1a of the present invention, shows a web of side panels 1a from which complimentary pairs of side panels 1a according to the present invention can be cut in a staggered configuration.

A sequence of fastening tabs 10 is attached to the web of side panels 1a shown in the lower part of Fig. 9a so that one of the distal or proximal edges 45, 46, respectively, of the fastening tabs 10 are arranged adjacent to a (hypothetical) cut line 150 in MD; Fig. 9a does not show which of such edges are arranged to the (hypothetical) cut line 150 so that the method provides side panels 1a where the gripping portions 20 and/or the fastening portions 30, respectively, face the (hypothetical) cut line as may be desirable. After a cut was applied along the MD cut line 150 -together with cuts along cut lines 151 in CD - to provide a sequence of complementary (left and right) pairs of individual side panels 1a. The side panels are reversed so that the two longitudinal edges 63 of the web - forming the proximal longitudinal edges of the side panels 1a - are facing each other; this is shown in the upper part of Fig. 9a. In this orientation, the pair of side panels can be attached, for example, to a web of rectangular chassis 1b of a diaper web from which a sequence of individual diapers may be obtained by applying CD cuts.

Fig. 9b shows an individual diaper obtained by the process of Fig. 9b. The diaper comprises a rectangular chassis 1b. The side panels of Fig. 9a are attached along the longitudinal edges of the diaper chassis so that the fastening tabs 10 are arranged at the rear portion of the diaper.

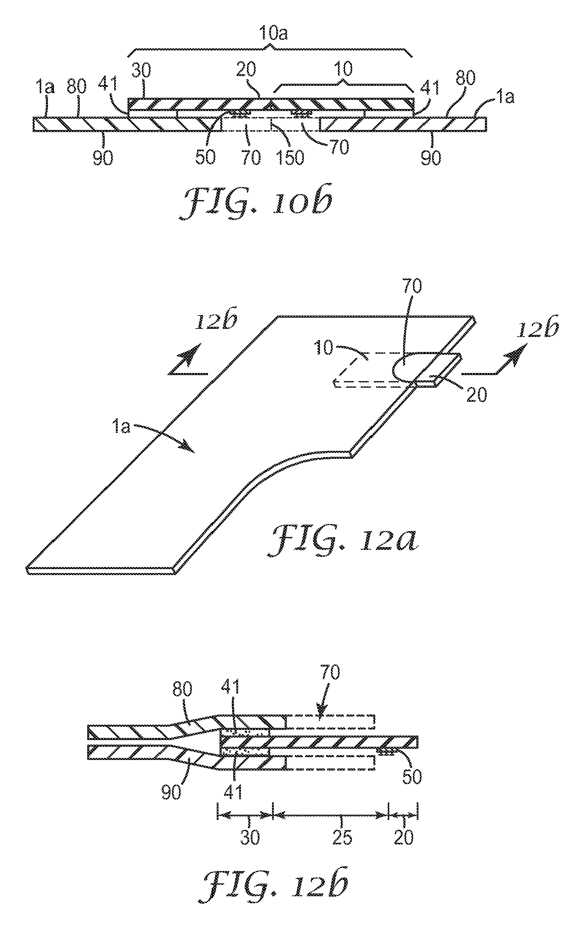

Fig. 10a which illustrates another method of making side panels 1a of the present invention, shows a web of side panels 1a from which complimentary pairs of side panels 1a according to the present invention can be cut. The web exhibits a (hypothetical) cut line 150 extending essentially along the center line of the web in MD. A sequence of cuts is applied in a first step along cut-lines 152 which are arranged normal and symmetrically relative to the (hypothetical) cut line 150 in regular intervals in MD. These cuts form a regular sequence of a pair of cut-out areas 70 in the web of side panels 1a. In the next step a fastening pad 10a comprising two fastening tabs 10 arranged so that their respective gripping portions 20 are facing each other and are flush with respect to each other, are laid upon the pairs of cut-out areas 70. The arrangement of the fastening pad 10a relative to the two cut-out areas 70 can be taken from the cross-sectional view of Fig. 10b applied along the line 10b-10b shown in Fig. 10a. The line where the two gripping portions 20 of the two fastening tabs 10 are abutting each other is arranged essentially flush with the (hypothetical) cut line 150. The two fastening tabs 10 of the fastening pad 10a are attached to the back sheet 80 of the web of side panels 1a at their respective fastening portions 30 via adhesive strips 41. The fastening tabs 10 of the fastening pad each comprises fastening means 50 which are exposed through the cut-out areas 70. The fastening tabs 10 are covering the respective cut-out area, i.e. the extension of the fastening tabs in MD is larger than the extension of the cut-out area in MD so that the fastening tab is supported in a single-sided configuration essentially along its extension in CD by the back sheet 80 of the side panel 1a. If the mechanical fastening means 50 extends over the full width of the fastening tab 10 in MD the mechanical fastening means may interact with the underlying back sheet 80 of the side panel so that a holding down effect is provided. The fastening pad 10a may be provided in one piece so that individual fastening tabs are obtained when applying a cut along the (hypothetical) cut line 150. When applying a cut along cut line 150 two sub-webs of side panels 1a are obtained. By reversing the orientation of these sub-webs relative to each other so that the two longitudinal edges of the web are facing each other, the sub-webs are provided in the correct orientation relative to a web of diaper chassis 1b. This is schematically indicated in the lower part of Fig. 10a. Individual side panels 1a are obtained from the sub-webs by applying cuts 51 in CD. The individual side panels 1a are attached to the web of diaper chassis 1b so that the areas close to the longitudinal proximal edges 61 of the side panel (forming the proximal edge areas of the side panels) are secured in the desired configuration to the areas close to the longitudinal edges 62 of the diaper chassis 1b.

Fig.10b is a cross-sectional view of the web of side panels 1a bearing a sequence of fastening pads 10a in their respective non-extended state along the line 10b-10b indicated in Fig. 10a.

Fig. 11a shows another embodiment of a side panel 1a of the present invention where a fastening tab 10 is sandwiched between the back sheet 80 and the top sheet 90, respectively, of the side panel so that in its non-extended state only the gripping portion 20 and part of the mechanical fastening means 50 (and thus part of the intermediate portion 25) are protruding beyond the longitudinal edge of the diaper. The fastening portion 30 of the fastening tab 10 is adhered with two adhesive strips 41 to the lower, non-exposed surfaces of the back sheet 80 and of the top sheet 90, respectively. The back sheet 80 and the top sheet 90 may be adhered along the longitudinal edges of the fastening tab 10 thereby forming a pocket sandwiched between the top sheet 90 and the back sheet 80, respectively, in which the intermediate portion 25 can be essentially freely extended when the fastening tab 10 is closed onto an absorbent article 1 the side panel 1a may be attached to (not shown). The mechanical fastening means 50 is facing the non-exposed surface of the top sheet 90 whereby a portion of the mechanical fastening means is protruding beyond the edge 60 of the side panel. Another portion of the mechanical fastening means 50 is in contact with and may interact with the non-exposed surface of the top sheet 90 so that the fastening tab 10 is held down in the non-extended state prior to use.

Fig.11b is a cross-sectional view of the web of the side panel 1a of Fig. 11a along the line 11b-11b indicated in Fig. 11a.

Fig. 12a shows another embodiment of a side panel 1a of the present invention the construction of which is similar to that of Fig. 11a but differs in that a cut-out section 70 was applied to the top sheet 90 and back sheet 80 to expose more of the intermediate portion in order to facilitate gripping of the fastening tab by the user. The fastening tab 10 is sandwiched between the back sheet 80 and the top sheet 90, respectively, of the side panel 1a so that in its non-extended state the gripping portion 20 and part of the mechanical fastening means 50 (and thus part of the intermediate portion 25) are protruding beyond the longitudinal edge 60 of the diaper extending beneath and above the fastening tab 10 and the cut-out portion. The fastening portion 30 of the fastening tab 10 is adhered with two adhesive strips 41 to the lower, non-exposed surfaces of the back sheet 80 and of the top sheet 90, respectively. The back sheet 80 and the top sheet 90 may be adhered along the longitudinal edges of the fastening tab 10 thereby forming a pocket sandwiched between the top sheet 90 and the back sheet 80, respectively, in which the intermediate portion 25 can be essentially freely extended when the fastening tab 10 is closed onto an absorbent article 1 the side panel may be attached to (not shown). The cut-out portion has a curved shape preferably proximally tapering to some extent so that the mechanical fastening means 50 which faces the non-exposed surface of the top sheet 90, partly contacts such surface of the top sheet 90. The interaction between the fastening means 50 and the non-exposed surface of the top sheet 90 preferably provides a holding down effect.

Fig.12b is a cross-sectional view of the web of the side panel 1a of Fig. 12a along the line 12b-12b indicated in Fig. 12a.

### List of reference numbers

- 1: absorbent article or side panel
- 1a: side panel
- 1b: chassis of absorbent article 1
- 2: rear portion of absorbent article
- 3: crotch portion of absorbent article
- 4: front portion of absorbent article
- 10: fastening tab
- 10a: fastening pad comprising two fastening tabs
- 20: gripping portion
- 25: intermediate portion
- 30: fastening portion
- 40: backing
- 41: adhesive
- 42: ultrasonic bonding points
- 45: distal edge of fastening tab
- 46: proximal edge of fastening tab
- 50: mechanical fastening means
- 51: male mechanical fastening element
- 60: distal longitudinal edge of side panel 1a
- 61: proximal longitudinal edge of side panel 1a
- 62: longitudinal edge of absorbent article
- 70: cut-out area
- 80: back sheet
- 90: top sheet
- 100: web of side panels 1a
- 101: longitudinal edge of web of side panels
- 150: cut-line in MD
- 151: cut-line in CD
- 152: cut-line for cut-out areas

## Claims

1. An absorbent article (1) comprising a top sheet (90) and a back sheet (80) and two opposite longitudinal edges (62) extending along the extension of the absorbent article (1) in machine direction , the absorbent article comprising at least one strip-shaped fastening tab (10, 10a) for securing said article on a wearer during use, wherein said fastening tab (10, 10a)
- comprises a fastening portion (30) through which it is attached to the absorbent article (1) and a gripping portion (20) which is opposite to the fastening portion (30),
- is elastically extendable or stretch-activatable to become elastically extendable, respectively,
- is foldless in the non-extended state, and
wherein less than 25 % of the extension of the fastening tab in CD in its non-extended state protrudes beyond or undershoots, respectively, the longitudinal edge (62) of the diaper.

2. An absorbent article (1) comprising a top sheet (90) and a back sheet (80) and two opposite longitudinal edges (62) extending along the extension of the absorbent article (1) in machine direction , the absorbent article comprising at least one strip-shaped fastening tab (10, 10a) for securing said article on a wearer during use, wherein said fastening tab (10, 10a)
- has a distal edge (45) and a proximal edge (46),
- comprises a fastening portion (30) through which it is attached to the absorbent article (1) and a gripping portion (20) which is opposite to the fastening portion (30),
- is elastically extendable or stretch-activatable to become elastically extendable, respectively,
- is foldless in the non-extended state, and
wherein the absolute value of the distance between the distal edge (45) of the fastening tab (10) and the longitudinal edge (62) of the diaper is less than 15 mm.

3. An absorbent article according to claim 2 wherein the distal edge (45) of the fastening tab (10) is arranged adjacent to a longitudinal edge (62) of the absorbent article(1).

4. An absorbent article according to any of the preceding claims wherein the fastening tab 10 is arranged in a single sided configuration essentially on the back sheet (80) or the top sheet (90) of the absorbent article (1), respectively.

5. An absorbent article according to any of claims 1 - 3 wherein the fastening tab 10 is arranged in a sandwich configuration between the top sheet (90) and the back sheet (80).

6. An absorbent article according to any of the preceding claims wherein the fastening tab (10) further comprises one or more mechanical fastening means (50).

7. An absorbent article according to any of the preceding claims wherein the absorbent article comprises a rear portion (2), a crotch portion (3) and a front portion (4) wherein the at least one fastening tab is attached to the rear portion(2).

8. An absorbent article according to any of the preceding claims wherein the top sheet and/or the back sheet of the absorbent article (1) have a distally open cut-out area (70) through which at least part of the non-protruding part of the fastening tab (10,10a) is exposed in its non-extended state.

9. An absorbent article according to any of the preceding claims wherein the fastening tab (10,10a) comprises an elastomeric or stretch-activatable film or layer.

10. An absorbent article according to claim 9 wherein the stretch-activatable layer or film comprises an elastic laminate with an elastic core and at least one skin layer being less elastic than the elastic core.

11. An absorbent article according to any of claims 9 or 10 wherein stretch-activatable layer or film is not pre-activated.

12. An absorbent article according to any of the preceding claims wherein the fastening portion (30) is essentially inelastic.

13. An absorbent article according to any of the preceding claims wherein the gripping portion (20) is essentially inelastic.

14. An absorbent article according to any of the preceding claims wherein the fastening tab (10,10a) comprises at least one nonwoven layer.

15. An absorbent article according to claim 13 wherein the mechanical fastening element (50) is arranged on the gripping portion (20).

16. An absorbent article according to any of the preceding claims wherein the extension of the fastening tab (10,10a) in CD is oriented substantially perpendicular to the longitudinal edge (60) of said absorbent article (1).

17. An absorbent article according to any of the preceding claims wherein the fastening portion (30) of the fastening tab (10,10a) is anchored to the back sheet (80) and/or top sheet (90) of the absorbent article (1), respectively, by adhesive bonding, thermal bonding, welding and/or ultrasonic bonding or a combination of these bonding means.

18. An absorbent article according to any of the preceeding claims, wherein the gripping portion (20) of the fastening tab (10,10a) comprises a fingerlift.

19. A side panel (1a) comprising a top sheet (90) and a back sheet (80), a distal longitudinal edge (60) and a proximal longitudinal edge (61) being attachable to the chassis (1b) of an absorbent article (1) which both are extending along the extension of the side panel (1a) in machine direction , the side panel (1a) comprising at least one strip-shaped fastening tab (10, 10a)
- which comprises a fastening portion (30) through which it is attached to the side panel (1a) and a gripping portion (20) which is opposite to the fastening portion (30), and which further comprises a mechanical fastening means (50),
- which is elastically extendable or stretch-activatable to become elastically extendable, respectively,
- which is not folded upon itself in the non-extended state, and
wherein less than 25 % of the extension of the fastening tab (10) in CD in its non-extended state protrudes beyond or undershoots, respectively, the distal longitudinal edge (60) of the side panel (1a).

20. A side panel (1a) comprising a top sheet (90) and a back sheet (80), a distal longitudinal edge (60) and a proximal longitudinal edge (61) being attachable to the chassis (1b) of an absorbent article (1) which both are extending along the extension of the side panel (1a) in machine direction , the side panel (1a) comprising at least one strip-shaped fastening tab (10, 10a)
- which comprises a fastening portion (30) through which it is attached to the side panel (1a) and a gripping portion (20) which is opposite to the fastening portion (30), and which further comprises a mechanical fastening means (50),
- which is elastically extendable or stretch-activatable to become elastically extendable, respectively,
- which is not folded upon itself in the non-extended state, and
wherein the absolute value of the distance between the distal edge (45) of the fastening tab (10) and the distal longitudinal edge (60) of the side panel (1a) is less than 15 mm.

21. A side panel (1a) according to any of claims 19 or 20 wherein the fastening tab 10 is arranged in a single sided configuration essentially on the back sheet (80) or the top sheet (90) of the side panel (1a), respectively.

22. A side panel (1a) according to any of claims 19 or 20 wherein the fastening tab 10 is arranged in a sandwich configuration between the top sheet (90) and the back sheet (80) of the side panel (1a).

23. A side panel (1a) according to any of claims 19 - 22 where the absolute value of the distance between the distal edge (45) of the fastening tab (10) and the distal longitudinal edge (60) of the side panel (1a) is less than 15 mm.

24. A side panel (1a) according to any of claims 19 - 22 wherein the distal edge (45) of the fastening tab (10) is arranged adjacent to the distal longitudinal edge (60) of the side panel (1a).

25. A side panel (1a) according to any of claims 19 - 23 wherein the fastening tab (10) further comprises one or more mechanical fastening means (50).

26. A side panel according to any of claims 19 - 24 wherein the top sheet (90) and the back sheet (80) are formed independently from each other by non-woven layers.

27. A continuous web (100) comprising a sequence of side panels (1a) according to any of claims 19 - 25.

28. A web according to claim 26 comprising a sequence of one or more complimentary pairs of side panels (1a) arranged next to each other in CD.

29. A roll of a continuous web of side panels (100) according to any of claims 26 to 27.

30. Method of manufacturing absorbent articles (1) according to any of claims 1 - 18, said comprising the steps of
(i) providing a web of absorbent articles (1a) comprising a top sheet (90) and a back sheet (80) and two opposite longitudinal edges (62) extending along the extension of the absorbent article (1) in machine direction ,
(ii) providing a sequence of individual fastening tabs (10, 10a) which each
- comprises a fastening portion (30) through which it can be attached to the absorbent article and a gripping portion (20) which is opposite to the fastening portion (30),
- is elastically extendable or stretch-activatable to become elastically extendable, respectively,
- is not folded upon itself in the non-extended state,
(iii) applying at least one fastening tabs (10,10a) to an absorbent article (1) of the web in a single-sided configuration or sandwich configuration, respectively, so that less than 25 % of its extension in CD protrudes in its non-extended state beyond the longitudinal edge (62) of the diaper, and
(iv) applying CD cuts to the web of absorbent articles to provide a sequence of individual absorbent articles each exhibiting at least one individual fastening tab (10,10a).

31. Method of manufacturing side panels (1a) according to any of claims 19 - 25 comprising the steps of
(i) providing a web of side panels (1a) comprising a top sheet (90) and a back sheet (80), a distal longitudinal edge (60) and a proximal longitudinal edge (61) being attachable to the chassis (1b) of an absorbent article (1) which both are extending along the extension of the side panel (1a) in machine direction,
(ii) providing a sequence of individual fastening tabs (10,10a) each of which
- comprises a fastening portion (30) through which it can be attached to the side panel (1a) and a gripping portion (20) which is opposite to the fastening portion (30),
- is elastically extendable or stretch-activatable to become elastically extendable, respectively,
- is not folded upon itself in the non-extended state,
(iii) applying at least one fastening tabs (10,10a) to a side panels (1a) of the web in a single-sided configuration or sandwich configuration, respectively, so that less than 25 % of its extension in CD protrudes in its non-extended state beyond the distal longitudinal edge (60) of the side panel, and
(iv) applying CD cuts to the web (100) of side panels (1a) to provide a sequence of individual side panels each exhibiting at least one individual fastening tab (10,10a).
